# EUROPEAN PATENT APPLICATION

(11) **EP 4 137 086 A2**
(43) Date of publication of application: **22.02.2023**
(21) Application number: 22190360.2
(22) Date of filing: 15.08.2022
(51) Int. Cl.: A61B 34/00, A61B 18/14, A61B 34/37, A61B 18/00, A61B 90/00

(54) **END EFFECTOR DRIVE MECHANISMS FOR SURGICAL INSTRUMENTS SUCH AS FOR USE IN ROBOTIC SURGICAL SYSTEMS**

(30) Priority: 16.08.2021 US 202117403504
(71) Applicant: Covidien LP, Mansfield, MA 02048 (US)
(72) Inventor: Tschudy, Christopher T., Boulder, 80301 (US); Kingsley, Dylan R., Boulder, 80301 (US); Malang, Sara A., Boulder, 80301 (US); Bodmer, James H., Boulder, 80301 (US); Moua, Tony G., Boulder, 80301 (US); Zeccola, Andrew W., Boston, 02210 (US); Siebenaller, Curtis M., Boulder, 80301 (US); Apostolopoulos, Haralambos P., Boulder, 80301 (US); Holbrook, Russell W., Boulder, 80301 (US); Whitney, William R., Boulder, 80301 (US); Weihe, Jason G., Boulder, 80301 (US); Heiliger, Zachary S., Boulder, 80301 (US)
(74) Representative: Maschio, Antonio

(57) **Abstract**

A robotic system includes an electrosurgical instrument having an instrument housing having a shaft with an end effector assembly and first and second jaw members attached thereto movable to grasp tissue. An input is configured to move the jaw members and is configured to operably couple to a torque sensor that measures the torque of the input during rotation thereof. A handle is remotely disposed relative to the instrument housing and is configured to communicate with the input for controlling the movement of the jaw members. A housing having a lever operably coupled thereto, houses components therein configured to operably connect to the input such that movement of the lever correlates to movement of the jaw members. The components are configured to regulate the resistance of the lever in response to the feedback from the torque sensor.

## Description

### FIELD

The present disclosure relates to surgical instruments and, more specifically, to end effector drive mechanisms for surgical instruments such as for use in robotic surgical systems.

### BACKGROUND

Robotic surgical systems are increasingly utilized in various different surgical procedures. Some robotic surgical systems include a console supporting a robotic arm. One or more different surgical instruments may be configured for use with the robotic surgical system and selectively mountable to the robotic arm. The robotic arm provides one or more inputs to the mounted surgical instrument to enable operation of the mounted surgical instrument.

A surgical forceps, one type of instrument capable of being utilized with a robotic surgical system, relies on mechanical action between its jaw members to grasp, clamp, and constrict tissue. Electrosurgical forceps utilize both mechanical clamping action and energy to heat tissue to treat, e.g., coagulate, cauterize, or seal, tissue. Typically, once tissue is treated, the tissue is severed using a cutting element. Accordingly, electrosurgical forceps are designed to incorporate a cutting element to effectively sever treated tissue. Alternatively, energy-based, e.g., thermal, electrical, ultrasonic, etc., cutting mechanisms may be implemented.

With traditional surgical instrumentation, e.g., open and endoscopic surgical instrumentation, the surgeon is typically able to feel or otherwise sense direct feedback from the end effector relating to the size of the tissue between the jaw members as well as the force being applied during manipulation and sealing. With robotic instrumentation, haptic feedback may be lost or sacrificed for ease of use to offset among other things, surgical fatigue.

### SUMMARY

As used herein, the term "distal" refers to the portion that is being described which is farther from an operator (whether a human surgeon or a surgical robot), while the term "proximal" refers to the portion that is being described which is closer to the operator. The terms "about," substantially," and the like, as utilized herein, are meant to account for manufacturing, material, environmental, use, and/or measurement tolerances and variations, and in any event may encompass differences of up to 10%. Further, to the extent consistent, any of the aspects described herein may be used in conjunction with any or all of the other aspects described herein.

Provided in accordance with aspects of the present disclosure is a robotic surgical system including an electrosurgical instrument including an instrument housing having a shaft extending therefrom. An end effector assembly is disposed at a distal end of the shaft and includes first and second jaw members movable between a first position wherein at least one of the jaw members is spaced relative to the other of the jaw members and a second position wherein the first and second jaw members cooperate to grasp tissue. An input is operably coupled to the instrument housing and is configured to move the jaw members between the first and second positions. The input is configured to operably couple to a torque sensor configured to measure the torque of the input during rotation thereof. One or more handles is remotely disposed relative to the instrument housing and is configured to communicate with the input for controlling the movement of the jaw members. The handle includes a housing having a lever operably coupled thereto, the housing defining a cavity therein configured to house one or more components configured to operably connect to the input such that movement of the lever relative to the housing correlates to movement of the jaw members between the first and second positions. The one or more components is configured to operably regulate the resistance of the lever in response to the feedback from the torque sensor.

In aspects according to the present disclosure, the one or more components are configured to operably regulate the resistance of the lever relative to a baseline torque measurement of the input measured during manufacturing. In other aspects according to the present disclosure, the one or more components are configured to operably regulate the resistance of the lever relative to a torque curve of the input.

In aspects according to the present disclosure, the correlation of the resistance of the lever to the torque of the input is linear. In other aspects according to the present disclosure, the correlation of the resistance of the lever to the torque of the input is non-linear.

In aspects according to the present disclosure, the combination of components disposed in the cavity include levers, gears, linkages, and springs.

Provided in accordance with aspects of the present disclosure is a robotic surgical system including an electrosurgical instrument including an instrument housing having a shaft extending therefrom. An end effector assembly is disposed at a distal end of the shaft and includes first and second jaw members having opposing tissue-contacting surfaces. The jaw members are movable between a first position wherein at least one of the jaw members is spaced relative to the other of the jaw members and a second position wherein the first and second jaw members cooperate to grasp tissue. The opposing tissue-contacting surfaces are operably connected to opposite polarities of the generator. An input is operably coupled to the instrument housing and is configured to move the jaw members between the first and second positions. One or more handles is remotely disposed relative to the instrument housing and is configured to communicate with the input for controlling the movement of the jaw members. The handle includes a housing having a lever operably coupled thereto. The housing includes a cavity defined therein configured to house one or more components therein configured to operably connect to the input such that movement of the lever relative to the housing correlates to movement of the jaw members between the first and second positions. Prior to the supplying electrosurgical energy to the opposing tissue-contacting surfaces, the generator is configured to sense an impedance between the opposing tissue contacting surfaces and determine an amount of tissue disposed therebetween. The one or more components are configured to operably regulate a resistance of the lever in response to the impedance feedback from the generator.

In aspects according to the present disclosure, the generator is configured to send a low energy signal across the opposing tissue-contacting surfaces to determine the presence of tissue and the corresponding impedance thereof disposed therebetween.

In aspects according to the present disclosure, the correlation of the resistance of the lever to the impedance is linear. In other aspects according to the present disclosure, the correlation of the resistance of the lever to the impedance is non-linear.

In aspects according to the present disclosure, the combination of components disposed in the cavity include levers, gears, linkages, and springs.

In aspects according to the present disclosure, the system further includes a switch operably disposed on the lever or the housing, the switch moveable between a first position where the lever, upon movement thereof, cooperates with the input to impart movement to the jaw members between the first and second positions with a closure pressure within the range of about 0.1 kg/cm² to about 2 kg/cm² and a second position wherein movement of the lever imparts movement to the jaw members with a closure pressure within the range of about 3 kg/cm² to about 16 kg/cm². In aspects according to the present disclosure, the resistance of the lever is configured to correlate with the position of the switch.

In aspects according to the present disclosure, the system further includes a tactile, visual and/or audible indicator of the closure pressure between the jaw members.

### BRIEF DESCRIPTION OF THE DRAWINGS

Various aspects and features of the present disclosure are described hereinbelow with reference to the drawings wherein:
FIG. 1 is a perspective view of a surgical instrument in accordance with the present disclosure configured for mounting on a robotic arm of a robotic surgical system;
FIG. 2 is a rear perspective view of a proximal portion of the surgical instrument of FIG. 1 with an outer housing removed;
FIG. 3 is a schematic illustration of an exemplary robotic surgical system configured to releasably receive the surgical instrument of FIG. 1;
FIG. 4A is a top perspective view of the robotic surgical system of FIG. 3 showing a pair of operating handles for remotely controlling the surgical instrument;
FIG. 4B is an enlarged view of one of the operating handles of the robotic surgical system of FIG. 4A; and
FIG. 5 is a enlarged view of the operating handles in accordance with another embodiment of the present disclosure.

### DETAILED DESCRIPTION

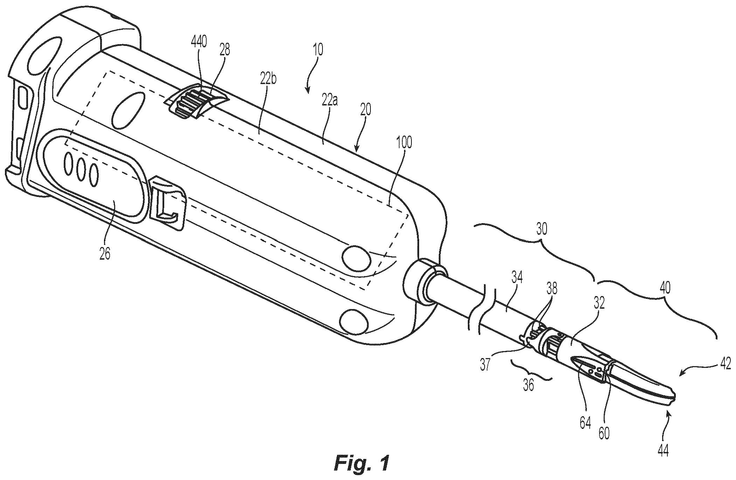

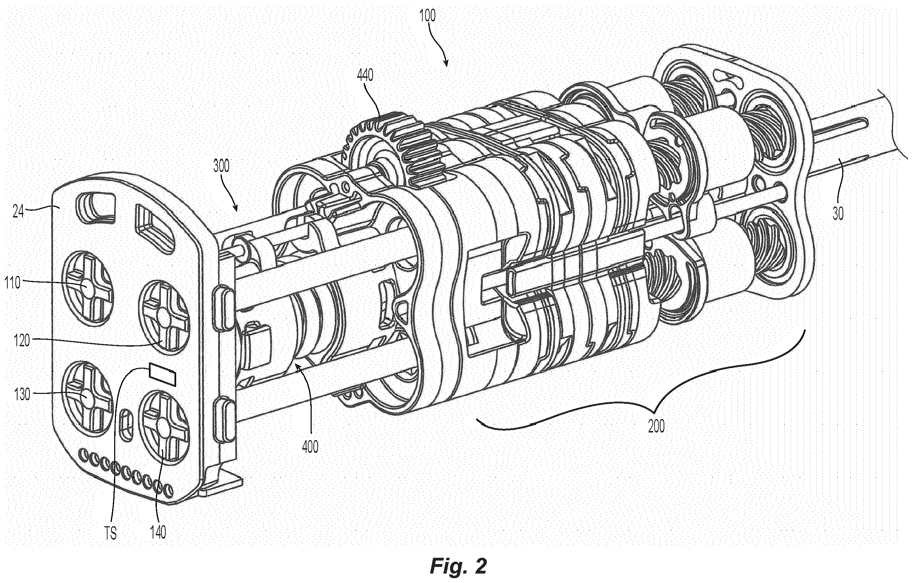

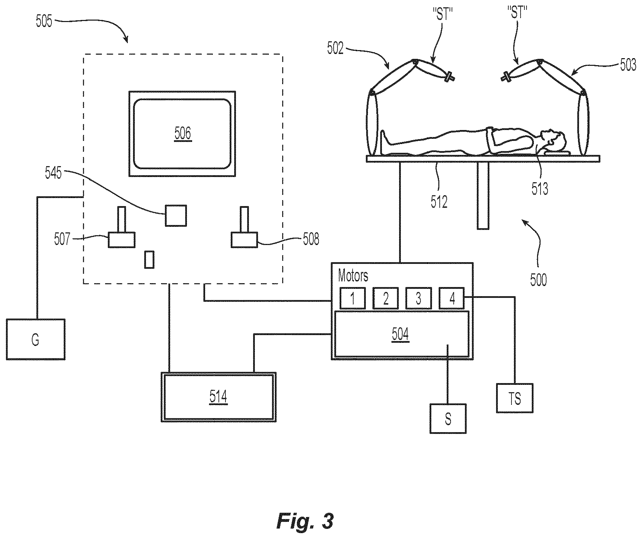

Referring to FIGS. 1 and 2, a surgical instrument 10 provided in accordance with the present disclosure generally includes a housing 20, a shaft 30 extending distally from housing 20, an end effector assembly 40 extending distally from shaft 30, and an actuation assembly 100 disposed within housing 20 and operably associated with shaft 30 and end effector assembly 40. Instrument 10 is detailed herein as an articulating electrosurgical forceps configured for use with a robotic surgical system, e.g., robotic surgical system 500 (FIG. 3). However, the aspects and features of instrument 10 provided in accordance with the present disclosure, detailed below, are equally applicable for use with other suitable surgical instruments (including non-robotic surgical instrument) and/or in other suitable surgical systems (including non-robotic surgical systems).

Housing 20 of instrument 10 includes first and second body portion 22a, 22b and a proximal face plate 24 (FIG. 2) that cooperate to enclose actuation assembly 100 therein. Proximal face plate 24 includes apertures defined therein through which inputs 110-140 of actuation assembly 100 extend. A pair of latch levers 26 (only one of which is illustrated in FIG. 1) extend outwardly from opposing sides of housing 20 and enables releasable engagement (directly or indirectly) of housing 20 with a robotic arm of a surgical system, e.g., robotic surgical system 500 (FIG. 3). An aperture 28 defined through housing 20 permits thumbwheel 440 to extend therethrough to enable manual manipulation of thumbwheel 440 from the exterior of housing 20 to permit manual opening and closing of end effector assembly 40.

Shaft 30 of instrument 10 includes a distal segment 32, a proximal segment 34, and an articulating section 36 disposed between the distal and proximal segments 32, 34, respectively. Articulating section 36 includes one or more articulating components 37, e.g., links, joints, etc. A plurality of articulation cables 38, e.g., four (4) articulation cables, or other suitable actuators, extends through articulating section 36. More specifically, articulation cables 38 are operably coupled to distal segment 32 of shaft 30 at the distal ends thereof and extend proximally from distal segment 32 of shaft 30, through articulating section 36 of shaft 30 and proximal segment 34 of shaft 30, and into housing 20, wherein articulation cables 38 operably couple with an articulation assembly 200 of actuation assembly 100 to enable selective articulation of distal segment 32 (and, thus end effector assembly 40) relative to proximal segment 34 and housing 20, e.g., about at least two axes of articulation (yaw and pitch articulation, for example). Articulation cables 38 are arranged in a generally rectangular configuration, although other suitable configurations are also contemplated.

With respect to articulation of end effector assembly 40 relative to proximal segment 34 of shaft 30, actuation of articulation cables 38 is effected in pairs. More specifically, in order to pitch end effector assembly 40, the upper pair of cables 38 is actuated in a similar manner while the lower pair of cables 38 is actuated in a similar manner relative to one another but an opposite manner relative to the upper pair of cables 38. With respect to yaw articulation, the right pair of cables 38 is actuated in a similar manner while the left pair of cables 38 is actuated in a similar manner relative to one another but an opposite manner relative to the right pair of cables 38.

Distal segment 32 of shaft 30 defines a clevis portion of end effector assembly 40 that supports first and second jaw members 42, 44, respectively. Each jaw member 42, 44 includes a proximal extension portion 43a, 45a and a distal body portion 43b, 45b, respectively. Distal body portions 43b, 45b define opposed tissue-contacting surfaces 46, 48, respectively. Proximal extension portions 43a, 45a are pivotably coupled to one another about a pivot pin 50 and are operably coupled to one another via a cam drive mechanism 52 (described in greater detail below) to enable pivoting of jaw member 42 relative to jaw member 44 and distal segment 32 of shaft 30 between a spaced-apart position (e.g., an open position of end effector assembly 40) and an approximated position (e.g., a closed position of end effector assembly 40) for grasping tissue between tissue-contacting surfaces 46, 48. As an alternative to this unilateral configuration, a bilateral configuration may be provided whereby both jaw members 42, 44 are pivotable relative to one another and distal segment 32 of shaft 30.

Opposing longitudinally-extending channels (not shown) are defined through tissue-contacting surfaces 46, 48, respectively, of jaw members 42, 44. A translating cutting element (not shown) is provided and selectively advanceable to enable cutting of tissue grasped between tissue-contacting surfaces 46, 48 of jaw members 42, 44, respectively. A cutting drive assembly 300 (FIG. 2) of actuation assembly 100 provides for selective actuation of cutting element through channel(s) of jaw members 42, 44 to cut tissue grasped between tissue-contacting surfaces 46, 48. Cutting drive assembly 300 is operably coupled to third input 130 of actuation assembly 100 such that, upon receipt of appropriate rotational input into third input 130, cutting drive assembly 300 advances the cutting element between jaw members 42, 44 to cut tissue grasped between tissue-contacting surfaces 46, 48.

Continuing with reference to FIGS. 1 and 2, a drive rod (not shown) is operably coupled to end effector assembly 40 such that longitudinal actuation of drive rod pivots jaw member 42 relative to jaw member 44 between the spaced-apart and approximated positions, as detailed below. More specifically, urging drive rod proximally pivots jaw member 42 relative to jaw member 44 towards the approximated position while urging drive rod distally pivots jaw member 42 relative to jaw member 44 towards the spaced-apart position. However, the reverse configuration is also contemplated. Drive rod extends proximally from end effector assembly 40 through shaft 30 and into housing 20 wherein drive rod is operably coupled with a jaw drive assembly 400 of actuation assembly 100 to enable selective actuation of end effector assembly 40 to grasp tissue therebetween and apply a closure force within an appropriate jaw closure force range, e.g., in response to an appropriate rotational input into fourth input 140.

Tissue-contacting surfaces 46, 48 of jaw members 42, 44, respectively, are at least partially formed from an electrically conductive material and are energizable to different potentials to enable the conduction of electrical energy through tissue grasped therebetween, although tissue-contacting surfaces 46, 48 may alternatively be configured to supply any suitable energy, e.g., thermal, microwave, light, ultrasonic, etc., through tissue grasped therebetween for energy-based tissue treatment. Instrument 10 defines conductive pathways extending through housing 20 and shaft 30 to end effector assembly 40 that may include lead wires, contacts, and/or electrically-conductive components to enable electrical connection of tissue-contacting surfaces 46, 48 of jaw members 42, 44, respectively, to an energy source (not shown), e.g., an electrosurgical generator via an electrosurgical cable extending therebetween, for supplying energy to tissue-contacting surfaces 46, 48 to treat, e.g., seal, tissue grasped between tissue-contacting surfaces 46, 48.

Actuation assembly 100 is disposed within housing 20 and includes articulation assembly 200, cutting drive assembly 300, and jaw drive assembly 400. Articulation assembly 200 is operably coupled between first and second inputs 110, 120, respectively, of actuation assembly 100 and articulation cables 38 such that, upon receipt of appropriate rotational inputs into first and/or second inputs 110, 120, articulation assembly 200 manipulates cables 38 (FIG. 1) to articulate end effector assembly 40 in a desired direction, e.g., to pitch and/or yaw end effector assembly 40. Cutting drive assembly 300, as noted above, enables reciprocation of the cutting element between jaw members 42, 44 to cut tissue grasped between tissue-contacting surfaces 46, 48 in response to receipt of appropriate rotational input into third input 130. Jaw drive assembly 400 is operably coupled between fourth input 140 of actuation assembly 100 and drive rod such that, upon receipt of appropriate rotational input into fourth input 140, jaw drive assembly 400 pivots jaw members 42, 44 between the spaced-apart and approximated positions to grasp tissue therebetween and apply a closure force within an appropriate closure force range.

Actuation assembly 100 is configured to operably interface with a robotic surgical system 500 (FIG. 3) when instrument 10 is mounted on robotic surgical system 500 (FIG. 3), to enable robotic operation of actuation assembly 100 to provide the above-detailed functionality. That is, robotic surgical system 500 (FIG. 3) selectively provides rotational inputs to inputs 110-140 of actuation assembly 100 to articulate end effector assembly 40, grasp tissue between jaw members 42, 44, and/or cut tissue grasped between jaw members 42, 44. However, it is also contemplated that actuation assembly 100 be configured to interface with any other suitable surgical system, e.g., a manual surgical handle, a powered surgical handle, etc. For the purposes herein, robotic surgical system 500 (FIG. 3) is generally described.

Turning to FIG. 3, a schematic representation of a robotic surgical system 500 is configured for use in accordance with the present disclosure. Aspects and features of robotic surgical system 500 not germane to the understanding of the present disclosure are omitted to avoid obscuring the aspects and features of the present disclosure in unnecessary detail.

Robotic surgical system 500 generally includes a plurality of robot arms 502, 503; a control device 504; and an operating console 505 coupled with control device 504. Operating console 505 may include a display device 506, which may be set up in particular to display three-dimensional images; and manual input devices or handles 507, 508, by means of which a person, e.g., a surgeon, may be able to telemanipulate robot arms 502, 503 in a first operating mode. Robotic surgical system 500 may be configured for use on a patient 513 lying on a patient table 512 to be treated in a minimally invasive manner. Robotic surgical system 500 may further include a database 514, in particular coupled to control device 504, in which are stored, for example, pre-operative data from patient 513 and/or anatomical atlases.

Each of the robot arms 502, 503 may include a plurality of members, which are connected through joints, and a mounted device which may be, for example, a surgical tool "ST." One or more of the surgical tools "ST" may be instrument 10 (FIG. 1), thus providing such functionality on a robotic surgical system 500.

Robot arms 502, 503 may be driven by electric drives, e.g., motors, connected to control device 504. Control device 504, e.g., a computer, may be configured to activate the motors, in particular by means of a computer program, in such a way that robot arms 502, 503, and, thus, their mounted surgical tools "ST" execute a desired movement and/or function according to a corresponding input from manual input devices 507, 508, respectively. Control device 504 may also be configured in such a way that it regulates the movement of robot arms 502, 503 and/or of the motors.

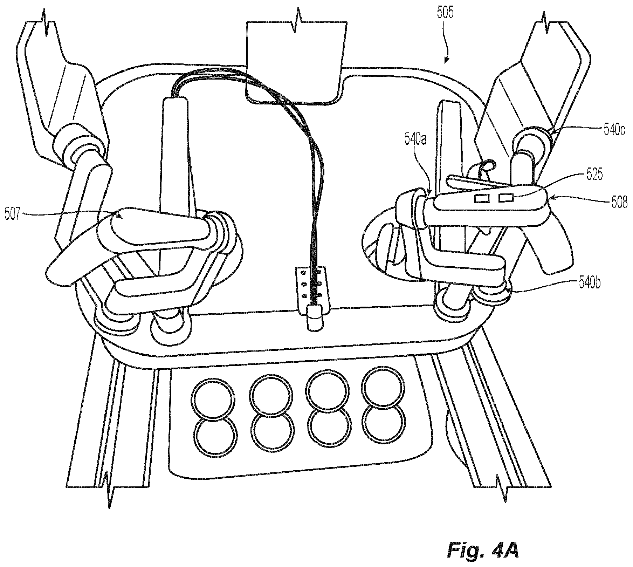

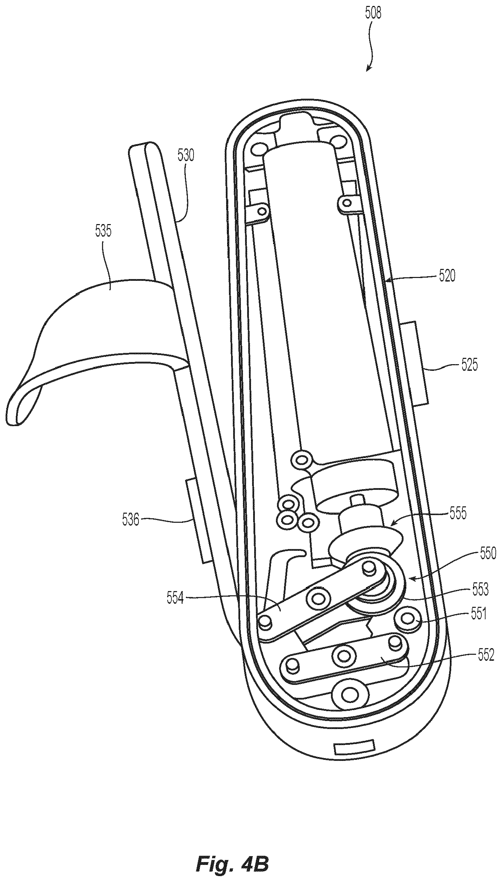

Turning now to FIGS. 4A and 4B, portions of the operating console 505 are depicted in more detail, namely, input devices or handles 507, 508. Each input device or handle 507, 508 includes similar components and, as such, only input device or handle 508 is depicted in FIG. 4B. Input device 508 includes a housing 520 defining a cavity 521 therein configured to house an actuation assembly 550 configured to communicate with one or more of the inputs 110-140 described in detail above. Housing 520 may be coupled to one or more gimbal mechanisms 540a-540c to control the extension, rotation, articulation, et. al. of the end effector assembly 40 as described above. Details relating to the various types of gimbal mechanisms that may be utilized for this purpose are disclosure in U.S. Patent Application Serial No. 16/306,420, filed November 30, 2018 entitled CONTROL ARM ASSEMBLIES FOR ROBOTIC SURGICAL SYSTEMS, the entire contents of which are incorporated by reference herein.

Actuation assembly 550 is operatively coupled to a lever 530 which, upon actuation thereof relative to housing 520, opens and closes jaw members 42, 44 of end effector assembly 40. More particularly, lever 530 couples to a series of components, e.g., links, couplers, and/or actuators 551-555, configured for communication with input 140 for controlling the closure of jaw members 42, 44 relative to one another as described above. Although depicted as a series of links, couplers and/or actuators 551-555, any type of mechanical or electromechanical arrangement is contemplated. Moreover, and as explained below, actuation of lever 530 relative to the actuation of the jaw members 42, 44 may be linear 1:1, non-linear 1:2 or variable during the entire range of movement or stroke of the lever 530.

Lever 530 includes a finger support 535 which is configured to facilitate relative movement of the lever 530 to the housing 520. More particularly, finger support 535 may be cuff-like to envelop one or more fingers of the user to promote retraction of the lever 530 away from the housing 520 and facilitate rotation of the housing about one or more of the above-identified gimbal mechanisms 540a-540c.

As mentioned above, during robotic surgery involving the sealing of vessels or tissue, it is important that the surgeon adequately control the pressure between the jaw members 42, 44 both when grasping and manipulating tissue and when clamping on a vessel or tissue to create a seal. As can be appreciated, the pressure between jaw members 42, 44 when delicately grasping and manipulating vessels or tissue is significantly less than when sealing vessels or tissue. The haptic feedback or "feel" of the jaw members 42, 44 is not always appropriately conveyed back the surgeon through the handles 508.

The various above-mentioned actuation assemblies, levers, gears, linkages, and springs discussed above all work in concert to facilitate actuation of the jaw members 42, 44 from the remote input devices or handles 507, 508. Typically, movement of the lever 530 a fixed distance relative to housing 520 will actuate the jaw members 42, 44 under the same force irrespective of the size of the vessel or tissue.

For example, with robotic vessel sealing devices such as those described above, the actuation of the jaw members 42, 44 is controlled through motor rotations which are mapped to the actuation lever 530. Since the forces required to generate a proper seal are typically on the order of 100 psi or greater, the software maps the relative movement of the surgeon's fingers and lever 530 to the housing 520 and correlates this relative movement to the jaw members 42, 44. Various software algorithms are utilized for this purpose. As a result, very little force is required to move the lever 530 relative to the housing 520 via the surgeon's fingers to generate the proper pressure for sealing tissue. This also helps to offset surgical fatigue. However, since the robotic sealer is designed to generate sealing pressures at the jaw members 42, 44 with far less comparative forces at the lever 530, in some instances too much pressure, e.g., pressures associated with sealing tissue, may be applied to a vessel or tissue bundle when the surgeon's intention is to simply grasp, manipulate or cut tissue.

In one embodiment according to the present disclosure, a torque sensor "TS" may be operably coupled to input 140 of instrument 10 or, alternatively, coupled to the motor control device 504. The torque sensor "TS", in turn, is operably coupled the one or more of the internal components 551-555 of drive assembly 550 of handle 508. Any changes in torque sensed by the torque sensor "TS" are converted into haptic feedback, e.g., resistance, in the lever 530. As can be appreciated and depending upon the sensitivity of the correlation of the torque sensor "TS" and the lever 530, the surgeon would be able to remotely "feel" differences in tissue, e.g., thickness, amount, etc., disposed between the jaw members 42, 44 and the relative closure pressure being applied thereto.

As mentioned above, actuation assembly 550 (and components 551-555) is operatively coupled to a lever 530 which, upon actuation, opens and closes jaw members 42, 44 of end effector assembly 40. The components 551-555 operably couple or are configured for communication with input 140 for controlling the closure of jaw members 42, 44 relative to one another as described above. Depending upon the actual feedback from toque sensor "TS", actuation of lever 530 relative to the actuation of the jaw members 42, 44 may be linear 1:1, non-linear 1:2 or variable during the entire range of movement or stroke of the lever 530. As can be appreciated, a variable correlation between the lever 530 and the jaw members 42, 44 may lessen the overall lever force required to generate the appropriate closure pressure at the jaw members 42, 44 for certain instrument functions, e.g., sealing tissue. For simple manipulation, grasping and cutting, the ratio of the lever force to jaw closure pressure may be linear 1:1 or even have a reverse ratio 2:1 depending upon a particular purpose. For sealing tissue, the ratios may be greater.

In embodiments, the torque sensor "TS' may need to be calibrated during manufacturing or during a so-called "homing" operation as new end effector assemblies 40 are interchanged. Details relating to various homing algorithms are disclosed in commonly-owned U.S. Patent Application Serial No. 63/183,089 entitled MOTOR POSITION CONTROL AND METHODS FOR ROBOTIC ASSISTED SEALING INSTRUMENT, the entire contents of which being incorporated by reference herein. As mentioned in therein, during manufacturing or a homing step, a baseline torque may be recorded (or a baseline torque curve could be recorded) in the torque sensor "TS", robotic controller 505 or control device 504 which is utilized by a software algorithm (or drive assembly 550 or one or more individual components 551-555) to adjust the lever 530 accordingly. As the torque sensor "TS" senses an increase in torque, the force required to actuate the lever 530 is adjusted accordingly, e.g., along a torque curve, depending upon the intended function of the instrument, e.g., grasp or seal.

A switch 536 may be operably coupled to handle 508 and utilized to convey the surgeon's intent to the software for allocating the correct haptic response. Alternatively, one or more activation switches 525 may be included on the handle 508 which supply electrosurgical energy to the jaw members 42, 44 for sealing tissue. Activation of one or more of the switches 525 may adjust the torque curve to correlate the resistive force of the lever 530 with a sealing pressure between the jaw members 42, 44. A delay may be instituted in the software algorithm to accomplish this purpose.

In envisioned embodiments, the software may include one or more "smart" features generally shown as smart feature "S" (FIG. 3) configured to distinguish normal variations in torque sensitivity of input 140 due to friction loss or offsets with moving components, articulation of shaft 30, repeated and normal wear and the like versus actual feedback as it relates to tissue. Smart features "S" may include a low-pass filter that attenuates the signals from the torque sensor "TS" to regulate the haptic response of lever 530 to more closely reflect actual tissue feedback.

As mentioned above, the jaw members 42, 44 include respective opposed tissue-contacting surfaces 46, 48 configured to receive electrosurgical energy of opposite potentials from an electrosurgical energy source, e.g., a generator "G" such that, when the generator "G" is activated, tissue disposed between the opposing tissue-contacting surfaces 46, 48 is treated. The type of tissue treatment or amount of electrosurgical energy (or both) may be dependent on the amount of tissue disposed between tissue-contacting surfaces 46, 48. For example, a low energy signal may be continuously sent across one or both tissue-contacting surfaces 46, 48 and the generator "G" may be configured to sense a "short" condition. If no tissue is disposed between the tissue-contacting surfaces 46, 48, the generator "G" will not sense a "short" condition and may prevent activation of the electrosurgical energy. On the other hand, if tissue is disposed between the tissue-contacting surfaces 46, 48, the tissue will cause a low energy "short" condition which is detected by the generator "G" freeing the generator "G" for activation via switch 525. The impedance level of the "short" may be measured and correlated to a look-up table or algorithm to determine how much tissue is disposed between the tissue-contacting surfaces 46, 48.

Once detected, the robotic controller 504 may be configured to provide feedback to the handle 508 to adjust the haptic feedback or resistance of the lever 535 according to the amount of tissue detected. As mentioned above, the surgeon would be able to remotely "feel" differences in tissue, e.g., thickness, amount, etc., disposed between the jaw members 42, 44 and the relative closure pressure being applied thereto. For simple manipulation, grasping and cutting, the ratio of the lever force to jaw closure pressure may be linear 1:1 or even have a reverse ratio 2:1 depending upon a particular purpose. For sealing tissue, the ratios may be greater. Moreover, and as explained above, actuation of lever 530 relative to the actuation of the jaw members 42, 44 may be non-linear 1:2 or variable during the entire range of movement or stroke of the lever 530.

Various visual or audible feedback 545 may be conveyed to the surgeon relating to the amount of tissue between the jaw members 42, 44 and the requisite pressure to be applied for the intended tissue action, e.g., manipulation, grasping, cutting or sealing. The feedback may be as simple as a "SAFE" signal when the surgeon intends to manipulate, grasp or cut tissue, e.g., green light, safe tone, or may be metered in relation to the amount of pressure being applied to tissue. Metering of the feedback either visually or audibly may provide feedback to the surgeon when he/she is approaching unintended pressures between the jaw members 42, 44 for these tissue actions. If the surgeon intends to seal tissue, the impedance level of the "short" may be measured and correlated to a look-up table or algorithm to determine how much tissue is disposed between the tissue-contacting surfaces 46, 48 to generate a proper tissue seal.

Switch 536 may be utilized to convey the surgeon's intent to the generator "G" for allocating the correct haptic response. In a first position, the lever 530, upon movement thereof, cooperates with the input to impart movement to the jaw members 42, 44 between the first and second positions with a closure pressure within the range of about 0.1 kg/cm² to about 2 kg/cm². Pressures up to and including the lower range of the below-identified sealing pressure range are also contemplated. In a second position, movement of the lever 530 imparts movement to the jaw members 42, 44 with a closure pressure within the range of about 3 kg/cm² to about 16 kg/cm².

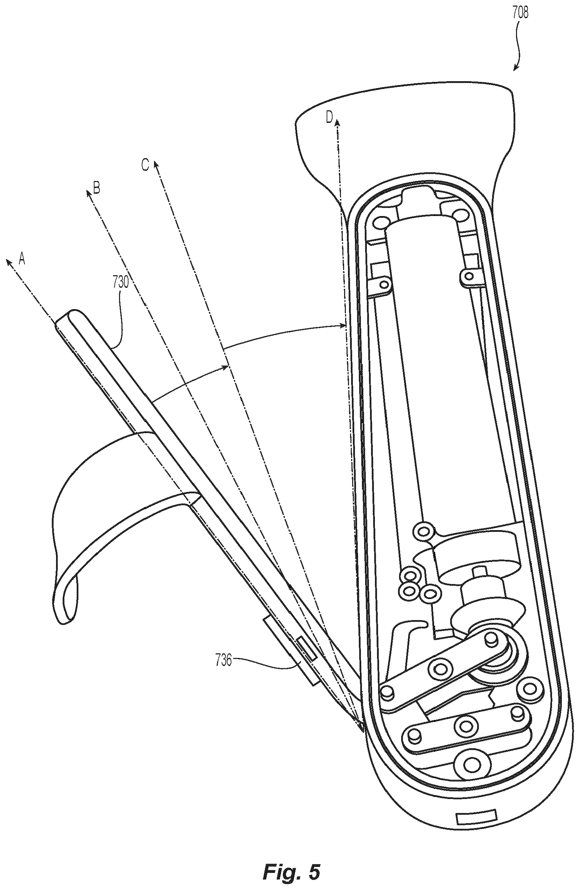

Turning briefly to FIG. 5, another embodiment of a handle 708 is envisioned which adjusts the haptic feedback of the lever 730 based on empirical data such that each lever stroke continues from a fully open position, through a typical grasping position and to a position for sealing tissue. The switch 736 may be configured to accommodate this purpose.

More particularly, point "A" position on the lever 730 indicates a fully open position wherein the jaw members 42, 44 are fully open to orient tissue therebetween. Between point "A" and point "B", with the lever 730 starting to close, there is only a slight haptic force associated with the lever 730, e.g., very little resistance on the lever 730. Point "B" may be customizable depending on the particular instrument or based on empirical data, e.g., point "B" is configured on the lever 730 as the point between a large vessel disposed within the jaws 42, 44 and nothing in the jaws 42, 44. Again, the switch 736 may be customized to accommodate this purpose. In other instances, point "B" may be customizable based on the feedback from sensors or torque.

Between point "B" and point "C" the lever 730 begins to compress a spring (or some other pressure control mechanism (not shown)) which will provide direct haptic feedback to the surgeon regarding pressure between jaw members 42, 44. The lever 730 and spring relationship may be linear or exponential or may be a combination, e.g., initially linear and then exponential. In embodiments, moving the lever between points "B" and "C" may induce a pressure within the above-identified sealing range and the surgeon may opt to seal tissue based on perceived haptic feedback within this stroke range. Alternatively, a point "C" may be included within the lever stroke that definitively identifies that the jaw pressure is within the sealing range. The movement of switch 736 may be customizable to effect the position of point "C" depending upon a particular purpose. Any further movement of the lever 730 does not increase the pressure between the jaw members 42, 44, e.g., the pressure is offloaded by the spring (or some other pressure control mechanism (not shown)) to not over-compress the tissue. Point "D" may be included as a bottom out point of the lever 730.

Various safety measures may be employed using one or more mechanical, electromechanical or software that would only allow a sealing pressure to be delivered when energy is being applied. For example, a slight delay may be programmed into the control software to delay activation of electrosurgical energy until a proper jaw pressure is applied between the jaw members 42, 44 via the lever 530.

It will be understood that various modifications may be made to the aspects and features disclosed herein. Therefore, the above description should not be construed as limiting, but merely as exemplifications of various aspects and features. Those skilled in the art will envision other modifications within the scope and spirit of the claims appended thereto.4

The invention may be described by reference to the following numbered paragraphs:-
1. A robotic surgical system, comprising:
   an electrosurgical instrument including an instrument housing having a shaft extending therefrom;
   an end effector assembly disposed at a distal end of the shaft, the end effector assembly including first and second jaw members movable between a first position wherein at least one of the jaw members is spaced relative to the other of the jaw members and a second position wherein the first and second jaw members cooperate to grasp tissue;
   an input operably coupled to the instrument housing and configured to move the jaw members between the first and second positions, the input configured to operably couple to a torque sensor configured to measure the torque of the input during rotation thereof; and
   at least one handle remotely disposed relative to the instrument housing and configured to communicate with the input for controlling the movement of the jaw members, the at least one handle including:
      a housing having a lever operably coupled thereto, the housing including a cavity defined therein configured to house one or more components therein configured to operably connect to the input such that movement of the lever relative to the housing correlates to movement of the jaw members between the first and second positions, the one or more components configured to operably regulate the resistance of the lever in response to the feedback from the torque sensor.
2. The robotic surgical system according to paragraph 1, wherein the one or more components are configured to operably regulate the resistance of the lever relative to a baseline torque measurement of the input measured during manufacturing.
3. The robotic surgical system according to paragraph 1, wherein the one or more components are configured to operably regulate the resistance of the lever relative to a torque curve of the input.
4. The robotic surgical system according to paragraph 1, wherein the correlation of the resistance of the lever to the torque of the input is linear.
5. The robotic surgical system according to paragraph 1, wherein the correlation of the resistance of the lever to the torque of the input is non-linear.
6. The robotic surgical system according to paragraph 1, wherein the combination of components disposed in the cavity include levers, gears, linkages, and springs.
7. A robotic surgical system, comprising:
   a generator configured to generate electrosurgical energy;
   an electrosurgical instrument including an instrument housing having a shaft extending therefrom;
   an end effector assembly disposed at a distal end of the shaft, the end effector assembly including first and second jaw members having opposing tissue-contacting surfaces, the jaw members movable between a first position wherein at least one of the jaw members is spaced relative to the other of the jaw members and a second position wherein the first and second jaw members cooperate to grasp tissue, the opposing tissue-contacting surfaces operably connected to opposite polarities of the generator;
   an input operably coupled to the instrument housing and configured to move the jaw members between the first and second positions; and
   at least one handle remotely disposed relative to the instrument housing and configured to communicate with the input for controlling the movement of the jaw members, the at least one handle including:
      a housing having a lever operably coupled thereto, the housing including a cavity defined therein configured to house one or more components therein configured to operably connect to the input such that movement of the lever relative to the housing correlates to movement of the jaw members between the first and second positions,
      wherein prior to the supplying electrosurgical energy to the opposing tissue-contacting surfaces, the generator is configured to sense an impedance between the opposing tissue contacting surfaces and determine an amount of tissue disposed therebetween and wherein the one or more components are configured to operably regulate a resistance of the lever in response to the impedance feedback from the generator.
8. The robotic surgical system according to paragraph 7, wherein the generator is configured to send a low energy signal across the opposing tissue-contacting surfaces to determine the presence of tissue and the corresponding impedance thereof disposed therebetween.
9. The robotic surgical system according to paragraph 7, wherein the correlation of the resistance of the lever to the impedance is linear.
10. The robotic surgical system according to paragraph 7, wherein the correlation of the resistance of the lever to the impedance is non-linear.
11. The robotic surgical system according to paragraph 7, wherein the combination of components disposed in the cavity include levers, gears, linkages, and springs.
12. The robotic surgical system according to paragraph 7, further comprising a switch operably disposed on at least one of the lever or the housing, the switch moveable between a first position where the lever, upon movement thereof, cooperates with the input to impart movement to the jaw members between the first and second positions with a closure pressure within the range of about 0.1 kg/cm² to about 2 kg/cm² and a second position wherein movement of the lever imparts movement to the jaw members with a closure pressure within the range of about 3 kg/cm² to about 16 kg/cm².
13. The robotic surgical system according to paragraph 12, wherein the resistance of the lever is configured to correlate with the position of the switch.

## Claims

1. A robotic surgical system, comprising:
an electrosurgical instrument including an instrument housing having a shaft extending therefrom;
an end effector assembly disposed at a distal end of the shaft, the end effector assembly including first and second jaw members movable between a first position wherein at least one of the jaw members is spaced relative to the other of the jaw members and a second position wherein the first and second jaw members cooperate to grasp tissue;
an input operably coupled to the instrument housing and configured to move the jaw members between the first and second positions, the input configured to operably couple to a torque sensor configured to measure the torque of the input during rotation thereof; and
at least one handle remotely disposed relative to the instrument housing and configured to communicate with the input for controlling the movement of the jaw members, the at least one handle including:
a housing having a lever operably coupled thereto, the housing including a cavity defined therein configured to house one or more components therein configured to operably connect to the input such that movement of the lever relative to the housing correlates to movement of the jaw members between the first and second positions, the one or more components configured to operably regulate the resistance of the lever in response to the feedback from the torque sensor.

2. The robotic surgical system according to claim 1, wherein the one or more components are configured to operably regulate the resistance of the lever relative to a baseline torque measurement of the input measured during manufacturing.

3. The robotic surgical system according to claim 1 or 2, wherein the one or more components are configured to operably regulate the resistance of the lever relative to a torque curve of the input.

4. The robotic surgical system according to claim 1, 2 or 3 wherein the correlation of the resistance of the lever to the torque of the input is linear.

5. The robotic surgical system according to any preceding claim, wherein the correlation of the resistance of the lever to the torque of the input is non-linear.

6. The robotic surgical system according to any preceding claim, wherein the combination of components disposed in the cavity include levers, gears, linkages, and springs.

7. A robotic surgical system, comprising:
a generator configured to generate electrosurgical energy;
an electrosurgical instrument including an instrument housing having a shaft extending therefrom;
an end effector assembly disposed at a distal end of the shaft, the end effector assembly including first and second jaw members having opposing tissue-contacting surfaces, the jaw members movable between a first position wherein at least one of the jaw members is spaced relative to the other of the jaw members and a second position wherein the first and second jaw members cooperate to grasp tissue, the opposing tissue-contacting surfaces operably connected to opposite polarities of the generator;
an input operably coupled to the instrument housing and configured to move the jaw members between the first and second positions; and
at least one handle remotely disposed relative to the instrument housing and configured to communicate with the input for controlling the movement of the jaw members, the at least one handle including:
a housing having a lever operably coupled thereto, the housing including a cavity defined therein configured to house one or more components therein configured to operably connect to the input such that movement of the lever relative to the housing correlates to movement of the jaw members between the first and second positions,
wherein prior to the supplying electrosurgical energy to the opposing tissue-contacting surfaces, the generator is configured to sense an impedance between the opposing tissue contacting surfaces and determine an amount of tissue disposed therebetween and wherein the one or more components are configured to operably regulate a resistance of the lever in response to the impedance feedback from the generator.

8. The robotic surgical system according to claim 7, wherein the generator is configured to send a low energy signal across the opposing tissue-contacting surfaces to determine the presence of tissue and the corresponding impedance thereof disposed therebetween.

9. The robotic surgical system according to claim 7 or 8, wherein the correlation of the resistance of the lever to the impedance is linear.

10. The robotic surgical system according to claim 7, 8 or 9 wherein the correlation of the resistance of the lever to the impedance is non-linear.

11. The robotic surgical system according to any one of claims 7 to 10 , wherein the combination of components disposed in the cavity include levers, gears, linkages, and springs.

12. The robotic surgical system according to any one of claims 7 to 11, further comprising a switch operably disposed on at least one of the lever or the housing, the switch moveable between a first position where the lever, upon movement thereof, cooperates with the input to impart movement to the jaw members between the first and second positions with a closure pressure within the range of about 0.1 kg/cm² to about 2 kg/cm² and a second position wherein movement of the lever imparts movement to the jaw members with a closure pressure within the range of about 3 kg/cm² to about 16 kg/cm².

13. The robotic surgical system according to claim 12, wherein the resistance of the lever is configured to correlate with the position of the switch.
